# EUROPEAN PATENT APPLICATION

(11) **EP 2 672 256 A1**
(43) Date of publication of application: **11.12.2013**
(21) Application number: 13170770.5
(22) Date of filing: 06.06.2013
(51) Int. Cl.: G01N 21/59, G01N 33/553

(54) **Sensor for detection of a target of interest**

(30) Priority: 06.06.2012 US 201261656354 P; 15.03.2013 US 201313835379
(71) Applicant: National Taiwan University, 10617 Taipei (TW)
(72) Inventor: Lin, Shiming, Taipei (TW); Lee, Si-Chen, Taipei 10617 (TW); Chang, Luan-Yin, Taipei (TW)
(74) Representative: Meyer-Dulheuer, Karl-Hermann

(57) **Abstract**

A sensor (100a) for detecting a target of interest (901) in a sample(900) comprises a container (101); a probe (113) disposed in the container and configured to bind to the target of interest; a circulation means (210, 220) configured to circulate substances in the container; a light source; a light receiver; a light selecting unit for allowing light of a predetermined wavelength to be received by the light receiver; and a detector configured to generate an electrical signal. The magnitude of the electrical signal reflects the amount of light that is received by the light receiver. The container is located between the light source and the light receiver and configured such that the probe (113) and the path of the light (112) emitted by the light source are in different regions inside the container.

## Description

### BACKGROUND OF THE DISCLOSURE

A biological sensor is an analytical device for detecting a target molecule by interaction of a biomolecule with the target molecule. Compared to culturing or polymerase chain reaction (PCR), a biological sensor can detect the existence of the target molecule within a relatively short time period. Some biological sensors use chromatographic immunoassay techniques. However, these chromatographic immunoassay-based biological sensors have adoption issues. For example, most chromatographic immunoassay-based biological sensors detect antibodies which are generated by the patients after a later stage of infection/disease.

### BRIEF DESCRIPTION OF THE DRAWINGS

Fig. 1A shows an illustrative embodiment of a sensor for detecting a target of interest (e.g., a biomolecule);
Fig. 1B shows another illustrative embodiment of a sensor for detecting a target of interest (e.g., a biomolecule);
Fig. 1C shows yet another illustrative embodiment of a sensor for detecting a target of interest (e.g., a biomolecule);
Fig. 2A shows an illustrative embodiment of a container of a sensor for detecting a target of interest (e.g., a biomolecule);
Fig. 2B shows another illustrative embodiment of a container of a sensor for detecting a target of interest (e.g., biomolecule);
Fig. 2C shows yet another illustrative embodiment of a container of a sensor for detecting a target of interest (e.g., a biomolecule);
Fig. 3 shows an illustrative embodiment of a sensor for detecting a target of interest (e.g., a biomolecule);
Fig. 4A shows an illustrative embodiment of a sensor for detecting a target of interest (e.g., a biomolecule);
Fig. 4B shows an illustrative embodiment of a sensor for detecting a target of interest (e.g., a biomolecule);
Fig. 4C shows an illustrative embodiment of a sensor for detecting a target of interest (e.g., a biomolecule);
Fig. 4D shows an illustrative embodiment of a sensor for detecting a target of interest (e.g., a biomolecule);
Fig. 5 shows an illustrative embodiment of a method for making a sensor;
Fig. 6A is a chart illustrating the signal strength during detection, by the sensor, of Enterovirus 71 in a 10% diluted sample collected from an infected patient;
Fig. 6B is a chart illustrating the signal strength during detection, by the sensor, of Enterovirus 71 in a 10% diluted sample collected from an infected patient;
Fig. 6C is a chart illustrating the signal strength during detection, by the sensor, of Enterovirus 71 in a control sample collected from a healthy person;
Fig. 7A is a chart illustrating the signal strength during detection, by the sensor, of Influenza A in a 10% diluted sample collected from an infected patient;
Fig. 7B is a chart illustrating the signal strength during detection, by the sensor, of Influenza A in a 10% diluted sample collected from an infected patient;
Fig. 7C is a chart illustrating the signal strength during detection, by the sensor, of Influenza A in a control sample collected from a healthy person;
Fig. 8A is a chart illustrating the signal strength during detection, by the sensor, of Influenza B in a 10% diluted sample collected from an infected patient;
Fig. 8B is a chart illustrating the signal strength during detection, by the sensor, of Influenza B in a 10% diluted sample collected from an infected patient; and
Fig. 8C is a chart illustrating the signal strength during detection, by the sensor, of Influenza B in a control sample collected from a healthy person, all arranged in accordance with embodiments of the disclosure.

### SUMMARY

Some embodiments of the present disclosure may generally relate to a sensor for detecting a target of interest. One exemplary sensor may comprise a container; a probe disposed in the container and configured to bind to a target of interest; a circulation means configured to circulate substances in the container; a light source; a light receiver; a light selecting unit for allowing light of a predetermined wavelength be received by the light receiver; and a detector configured to generate an electrical signal. The magnitude of the electrical signal reflects the amount of light that is received by the light receiver. The container is located between the light source and the light receiver. Moreover, the container is configured such that the probe and the path of the light emitted by the light source are in different regions inside the container.

Some additional embodiments of the present disclosure may generally relate a sensor for detecting a target of interest. One exemplary sensor may comprise a container; a probe disposed in the container and configured to bind to a target of interest; a circulation means configured to circulate substances in the container; a light source that emits light of a predetermined wavelength; a light receiver; and a detector configured to generate an electrical signal. The magnitude of the electrical signal reflects the amount of light that is received by the light receiver. The container is located between the light source and the light receiver, and the container is configured such that the probe and the path of the light emitted by the light source are in different regions inside the container.

The foregoing summary is illustrative only and is not intended to be in any way limiting. In addition to the illustrative aspects, embodiments, and features described above, further aspects, embodiments, and features will become apparent by reference to the drawings and the following detailed description.

### DETAILED DESCRIPTION

In the following detailed description, reference is made to the accompanying drawings, which form a part hereof. In the drawings, similar symbols typically identify similar components, unless context dictates otherwise. The illustrative embodiments described in the detailed description, drawings, and claims are not meant to be limiting. Other embodiments may be utilized, and other changes may be made, without departing from the spirit or scope of the subject matter presented here. It will be readily understood that the aspects of the present disclosure, as generally described herein, and illustrated in the figures, can be arranged, substituted, combined, and designed in a wide variety of different configurations, all of which are explicitly contemplated and make part of this disclosure.

In this disclosure, the term "probe" generally refers to a substance (e.g., a biomolecule) that is capable of binding to a target of interest (e.g., a biomolecule). For example, a probe may have a binding affinity for the target of about 100 piconewtons (pN) to about 500 pN. Nonlimiting examples of probes include antibodies, antibody fragments that retain the ability to bind to the target of interest, nucleic acids (e.g., DNA, RNA, aptamers), antigens, and enzymes. In a sensor as described herein, a single probe that recognizes a single target of interest, or two or more probes that recognize a single target or multiple targets of interest may be used.

The term "target" generally refers to any molecule that is detectable with a sensor as described herein. A target may include, but is not limited to, a biomolecule. Examples of targets that are detectable in the sensors described herein include, but are not limited to, biomolecules (for example, virus, proteins, nucleic acids, carbohydrates, lipids), and other types of molecules (e.g., small molecules) such as, haptens, and toxins. In some embodiments, the target is a biomolecule that is present in a bodily fluid and/or tissue.

In some embodiments, a sensor for detecting a target of interest (e.g., a biomolecule) includes a light source, a container, a circulation means for circulating substances inside the container, and a light receiver having a light detector that generates an electrical signal that is proportionate to the amount of light received by the light receiver. At least one inner surface of the container includes probes immobilized on a material that is disposed on the container surface. The light source is configured to generate light that passes through a predetermined region in the container and eventually to the light detector. The predetermined region in the container which the light is configured to pass through is a space in the container that does not overlap the probes. The light may be of a specific wavelength. Depending on the target to be detected, the specific wavelength may be changed accordingly. The specific wavelength may be determined by any technical feasible approaches. In some embodiments, the specific wavelength is determined by scanning the target with the visible light spectrum or UV light spectrum. The maximum absorption wavelength of the target in the visible light spectrum may be the specific wavelength. For example, any of enterovirus 71, influenza A virus, and influenza B virus has a maximum absorption at about 560 nanometer (nm) wavelength in the visible light spectrum and has a relatively greater absorption at about 280 nm wavelength in the ultra-violet spectrum. Adenovirus has a maximum absorption at about 340 nm wavelength in the visible light spectrum and has a relatively greater absorption at about 280 nm wavelength in the ultra-violet spectrum.

In some embodiments, the light source may generate visible light or ultra-violet light. A light filter may be placed between the light source and the container so that the light entering the container has a specific wavelength. Alternatively, the light filter may be placed between the container and the light detector so that the light entering the light detector has a specific wavelength. Alternatively, some optical elements (e.g., slit, grating, mirror and a linear charge-coupled device) may be placed between the container and the light detector to produce monochromatic light having a specific wavelength from the visible light that passed through the container, and allow the monochromatic light to enter the light detector. Alternatively, the light source may be a monochromatic light source.

In some embodiments, the container includes a substrate, a material disposed on the substrate and one or more probe(s) disposed on the material. The probe is immobilized on the material. The probe may be a biological substance, for example, an antibody, an antibody fragment, a nucleic acid, an aptamer, an antigen or an enzyme, or any substance that is capable of binding to a target of interest. The material is compatible with the probe and the probe can be immobilized on the material. The material may be, for example, a metal such as gold, silver, copper and nickel. The substrate may be composed of any composition that is technically feasible for the material to be disposed thereon, and that does not interfere with detection of a target of interest as described herein. Some examples of suitable substrates may include, without limitation, glass, metal, silicon or polymers.

The material may include a pattern configured to increase the surface area available for the deposition of the probe. In some embodiments, the pattern may be a film coated on the substrate. In some other embodiments, the coated film may be annealed. The annealing makes the surface of the coated film uneven. The uneven surface may provide an increase in the surface area available for the deposition of the probe.

In some embodiments, the pattern may be a metal rod array disposed on the substrate. The three dimensional property of the metal rod array may also provide an increase in the surface area available for the deposition of the probe.

The probe is disposed or immobilized on the material through one or more chemical bonds (e.g., covalent bond) with the material. The probe may form a "lock and key" relationship with the target to be detected by the sensor. For example, the probe may be DNA, RNA, a protein, an antibody, an antibody fragment, an aptamer, an antigen, or an enzyme. In some embodiments, the probe is an antibody and the target is an antigen to which the antibody binds.

The circulation means is configured to circulate the substances in the container, so as to propel the targets, if any, towards the probe. In some embodiments, the circulation means may include a first magnet disposed in the container, and a second magnet coupled with a motor disposed outside the container, near a wall of the container. When the motor drives the second magnet to spin, the first magnet in the container will also spin. In another example, the circulation means may include a propelling means attached to a wall inside the container, and electrically connected to a motor configured to drive the propelling means.

A sample potentially including the target is introduced into the sensor and then flows over the probe. If the target exists in the sample, the amount of photons passing through the predetermined region in the container before the circulation means circulates the substance in the container will be different than the amount of photons passing through the predetermined region in the container after the circulation means circulated the substance in the container because the target would couple with the probes immobilized on the material that is disposed on the container surface. If the target does not exist in the sample, the amount of photons passing through the predetermined region in the container will remain substantially the same because the density of the sample in the predetermined region of the container before and after the circulation means circulated the substances in the container does not change.

The probes and the path of the light emitted by the light source are in different regions inside the container. In some embodiments, the probes are disposed in the upper portion of the container, while the predetermined region which the light passes through the container may be in the lower portion of the container where no probes are disposed. In some other embodiments, the predetermined region which the light passes through the container may be in the portion of the container where the probes are also disposed, but does not overlap the probes.

In some embodiments, a method for making a sensor is disclosed. The method includes providing a material which includes a pattern configured to increase the surface area available for binding with the probes, disposing the material on a substrate and disposing the probes configured to interact with the target of interest on the material.

In some embodiments, the material is a film. The substrate may be cleaned before the material is disposed on the substrate. In some embodiments, before the material is disposed on the substrate, an adhesion layer is disposed on the substrate first. Then the material is disposed on the adhesion layer. The adhesion layer may be chromium.

In some other embodiments, the material is an annealed film which has an uneven surface pattern. The material may be annealed at a temperature from about 300 degrees Celsius (°C) to about 500 °C after the material is disposed on the adhesion layer.

In some other embodiments, the material includes a rod array pattern. A photoresist is coated on the substrate and a photolithography process is performed to form the rod array. The size of each rod in the rod array and the distance between two adjacent rods are associated with the size of the probe and the size of the target of interest.

In some embodiments, a method for detecting a target of interest with a sensor described herein is disclosed. The sensor includes a light source, a container, a circulation means and a light detector. The container includes a substrate, a material disposed on the substrate and probes configured to interact with the target of interest. The probes are disposed and immobilized on the material. The light source is configured to generate light that passes through a predetermined region in the container and eventually to the light detector. The predetermined region in the container which the light is configured to pass through is a space in the container that does not overlap the probes. The method includes placing a sample that potentially includes the target of interest in the sensor and transmitting light from the light source through the predetermined region in the container and obtaining a first signal based on the light received by a light detector of the sensor.

The method also includes actuating the circulation means for a predetermined amount of time to propel substances in the container towards the probes and obtaining a second signal based on the light received by the light detector after the predetermined amount of time. The method further includes comparing the first signal and the second signal and determining whether the target exists in the sample based on the comparison.

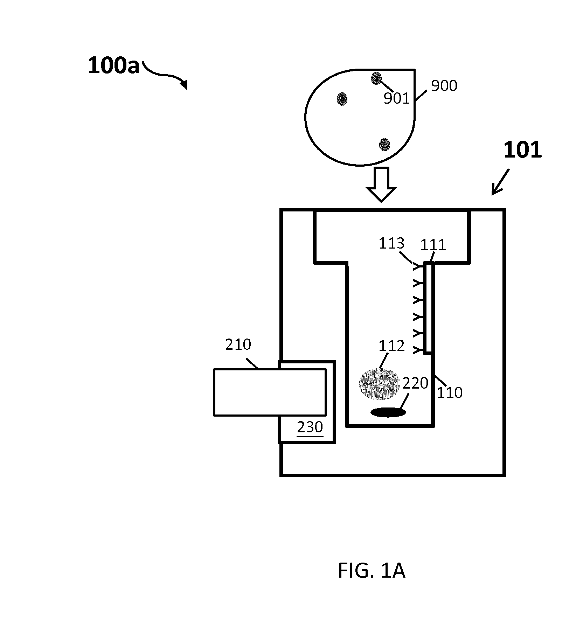

FIG. 1A is an illustrative embodiment of a sensor 100a for detecting a target of interest. The sensor 100a includes a container 101. The container 101 forms an apparatus for binding a target of interest 901, and includes a material 111 disposed on a substrate 110 (e.g., one or more walls of the container) and probes 113 that are capable of binding to the target 901 disposed on the material 111. The probes 113 are disposed in an upper portion of the container 101. A light 112 is transmitted from a light source of the sensor 100a to a light receiver of the sensor 100a through a lower portion of the container 101. The light source is configured such that the path of the light emitted by the light source is away from the probes 113, so that the light passing through the container 101 does not come in contact with the probes 113 or the target of interest 901 bound to the probes 113.

The sensor 100a further includes a circulation means 210, 220 configured to circulate substances in the container 101. In some embodiments, the circulation means 210, 220 may include a first magnet 220 freely disposed in the container 101 and a motor 210 disposed outside the container 101, near a wall of the container 101. The motor 210 may include a second magnet (not shown), and when the motor 210 works to drive the second magnet to spin, the second magnet in the motor 210 may cause the first magnet 220 in the container 101 to spin simultaneously. Alternatively, the circulation means 210, 220 may include a propelling means attached to a wall inside the container 101 and electrically connected to a motor configured to drive the propelling means.

The container 101 may further comprise a recess 230 configured to fit the motor 210, and thus decrease the size of the sensor. In the case where the circulation means 210, 220 include the first magnet 220 disposed inside the container 101, with the recess 230, the power required to actuate the first magnet 220 by the motor 210 may be optimized, because the distance between the first magnet 220 and the motor 210 is minimized.

The sensor 100a further includes a solution that does not contain the target, such as a buffer solution, in the container 101. A sample 900 potentially including the target of interest 901 is introduced into the container 101. The light 112 is then configured to pass through the container 101 in a first time slot and is received by the light receiver. During the first time slot, the substances in the sample 900 sinks to the lower portion of the container 101 where the light 112 is configured to pass through. The light receiver further includes a photodiode detector configured to generate a first electronic signal based on the amount of the light 112 that is received by the light receiver in the first time slot. The light receiver further includes a processor for processing the first electronic signal.

The circulation means 210, 220 is actuated to circulate the substances in the container for a predetermined amount of time. By the circulation, the substances in the lower portion of the container 101 are propelled to the upper portion of the container 101, and the target of interest 901 may bind to the probes 113 on the material 111. After the predetermined period of time has lapsed, the circulation means 210, 220 is stopped, and the light 112 is configured to pass through the container 101 in a second time slot and is received by the light receiver. The photodiode detector of the light receiver is configured to generate a second electronic signal based on the amount of the light that is received by the light receiver in the second time slot. The processor of the light receiver is configured to further process the second electronic signal. If the magnitude of the second signal is different from that of the first signal, the processor determines that the target of interest exists in the sample. On the other hand, if the magnitude of the second signal is substantially the same as that of the first signal, the processor determines that the target of interest does not exist in the sample.

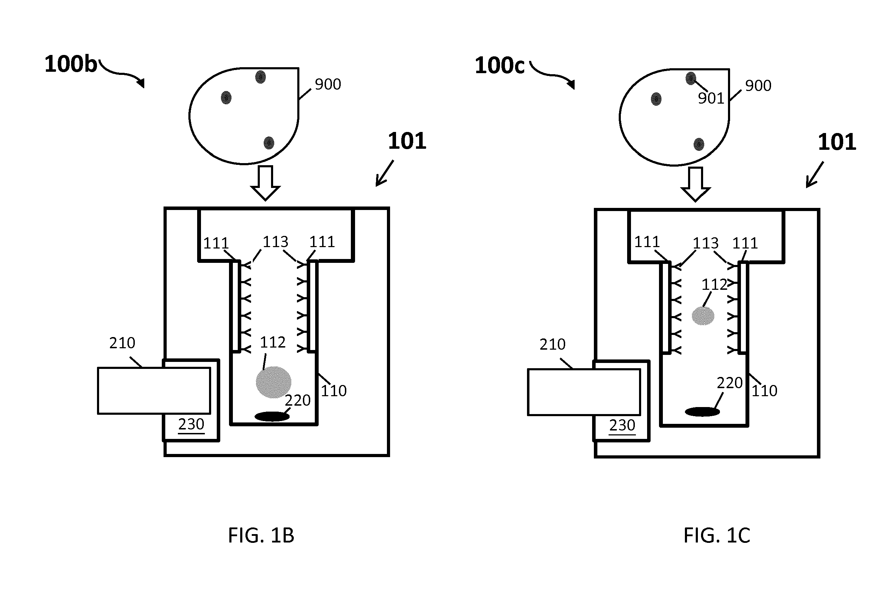

FIG. 1B is an illustrative embodiment of a sensor 100b for detecting a target of interest. The sensor 100b in FIG. 1B is similar to the sensor 100a in FIG. 1A, except the material 111 and the probes 113 are disposed on more than one walls of the container 101. With the additional probes 113 in the container 101, the amount of time which the circulation means 210, 220 needs to be actuated may be shortened, because there exit more probes 113 readily available to bind with the target of interest 901.

FIG. 1C is an illustrative embodiment of a sensor 100c for detecting a target of interest. The sensor 100c in FIG. 1C is similar to the sensor 100b in FIG. 1B, except the light 112 is configured to pass through a region that is in the same portion of the container 101 as the probes 113. The light 112 is configured to avoid overlapping with the probes 113, so as to prevent scattering of the light 112 by the probes 113 or targets 901 bound to the probes 113.

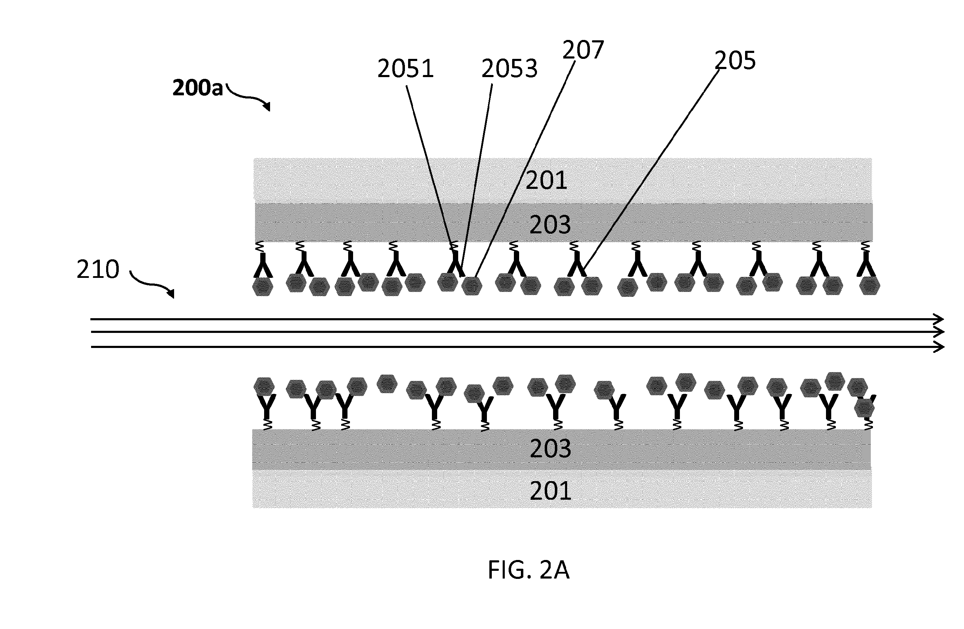

Fig. 2A shows an illustrative embodiment of an apparatus for binding a target of interest. The apparatus 200a includes a substrate 201, a film 203 disposed on the substrate and probes 205 disposed on the film 203. The thickness of the film 203 may be, for example, a substantially even thickness of about 5nm to about 200nm. A first surface 2051 of the probe 205 is disposed on the material 203. A second surface 2053 of the probe 205 having a binding affinity for the target 207 is configured such that it is available to couple with the target 207 when the target 207 is present.

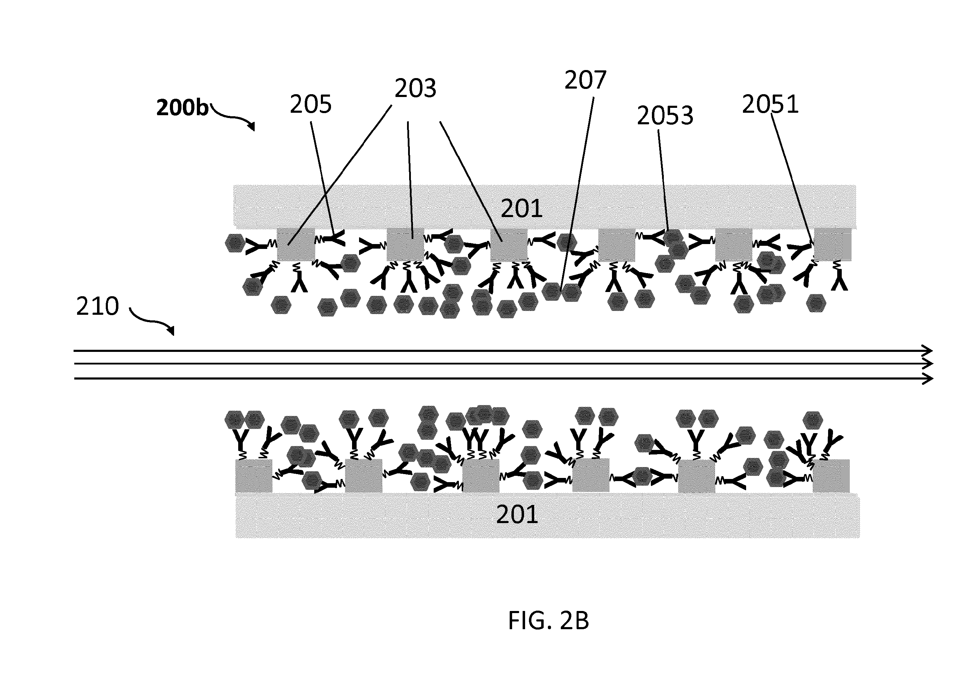

Fig. 2B shows an illustrative embodiment of an apparatus for binding a target of interest. The apparatus 200b includes a substrate 201, a rod array 203 disposed on the substrate 201 and probes 205 disposed on the rod array 203. The width and the length of any rod in the rod array 203 and the distance between two adjacent rods are associated with the size of the probe 205 and the target of interest 207. For example, the rod of the rod array may have a length from 200 to 900 nm, a width from 200 to 900 nm and a height from 15 to 1500 nm. The distance between each rod in the rod array may be from 200 to 900nm. In some embodiments, the rod of the rod array may have a length of approximately 500 nm, a width of approximately 500 nm and a height of about 100 nm, and the distance between each rod may be approximately 500 nm. A first surface 2051 of the probe 205 is disposed on the rod array 203. A second surface 2053 of the probe 205 having a binding affinity for the target 207 is configured such that it is available to couple with the target 207 when the target 207 is present.

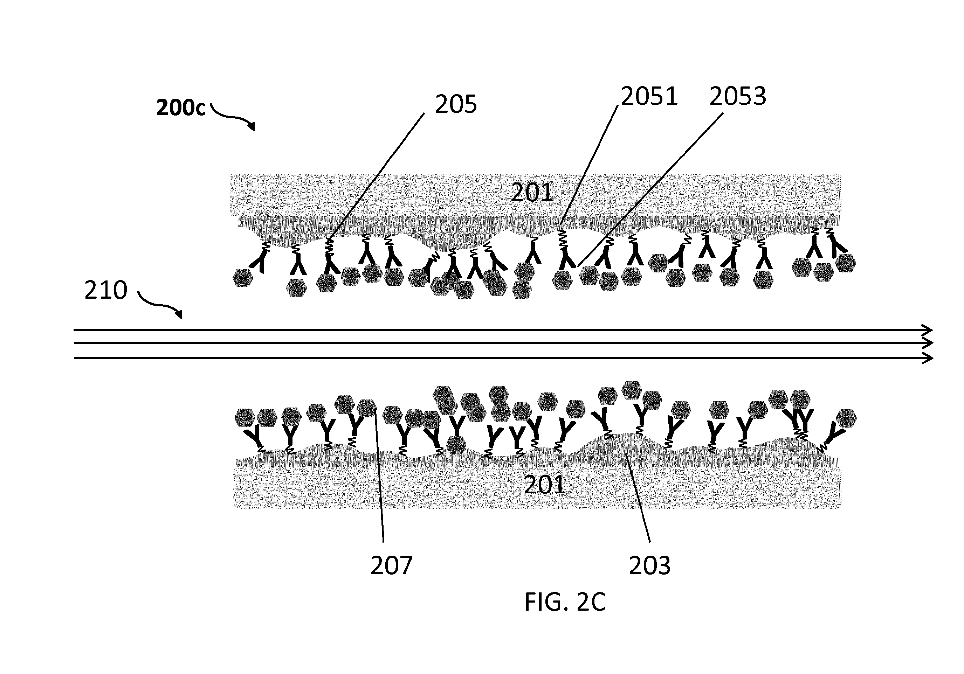

Fig. 2C shows an illustrative embodiment of an apparatus for binding a target of interest. The apparatus 200c includes a substrate 201, a film 203 disposed on the substrate and probes 205 disposed on the film 203. The film 203 has an uneven thickness. In some embodiments, the film may be first coated on the substrate 201 and then annealed to form the uneven thickness. In some embodiments, the thickness of the film 203 may vary from approximately 0.5 nm to approximately 30 nm. A first surface 2051 of the probe 205 is disposed on the material 203. A second surface 2053 of the probe 205 having a binding affinity for the target 207 is configured such that it is available to couple with the target 207 when the target 207 is present.

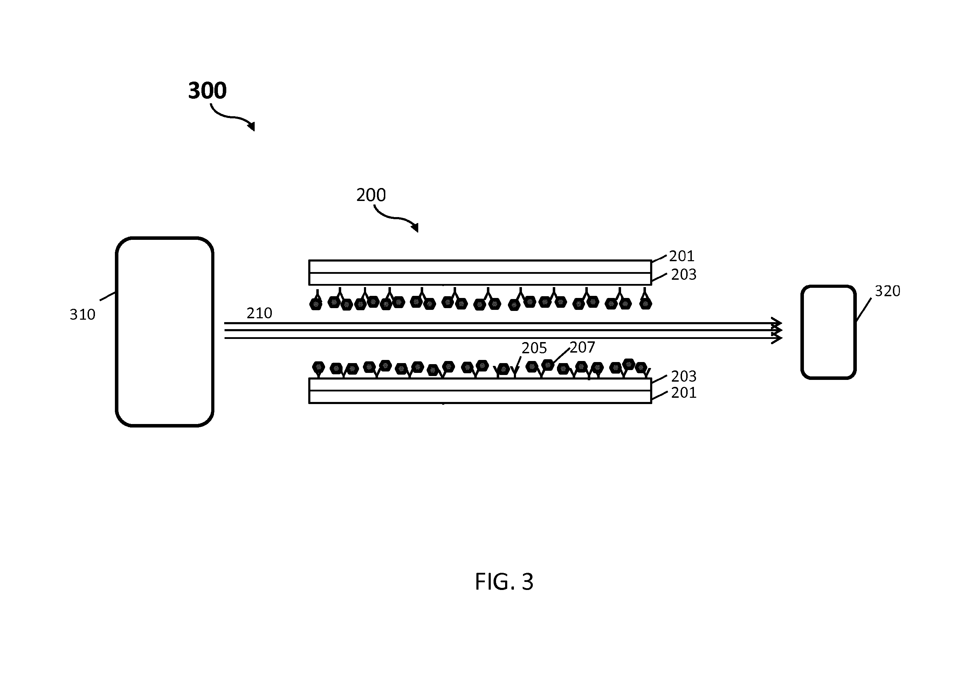

FIG. 3 shows an illustrative embodiment of an apparatus 300 for providing and detecting light passing through an apparatus 200 for detecting a target of interest. The apparatus 300 includes a light source 310 and a light receiving unit 320. The apparatus 200 for detecting a target of interest is disposed between the light source 310 and the light receiving unit 320.

The apparatus 200 for detecting a target of interest may include a substrate 201, a film 203 disposed on the substrate and a probe 205 disposed on the film 203. The configuration of the substrate 201, the film 203 and the probe 205 in the apparatus 200 for detecting a target of interest may be the same or similar to those in the apparatus 200 illustrated and described in reference to FIGs. 2A, 2B and 2C.

Light 210 (shown as arrows in FIG.3) from the light source 310 pass through the apparatus 200 for detecting a target of interest, and is received by the light receiving unit 320.

In some embodiments, the light source 310 may generate visible light or ultra-violet light. A light filter may be placed between the light source 310 and the apparatus 200 for detecting a target of interest so that the light 210 entering the apparatus 200 for detecting a target of interest has a specific wavelength. Alternatively, the light filter may be placed between the apparatus 200 for detecting a target of interest and the light receiving unit 320 so that the light 210 entering the light receiving unit 320 has a specific wavelength. Alternatively, some optical elements (e.g., slit, grating, mirror and a linear charge-coupled device) may be placed between the apparatus 200 for detecting a target of interest and the light receiving unit 320 to make the visible light (or ultra-violet light) passed through the apparatus turn to a monochromatic light with a specific wavelength before entering the light receiving unit 320. The light source 310 may also be a monochromatic light source.

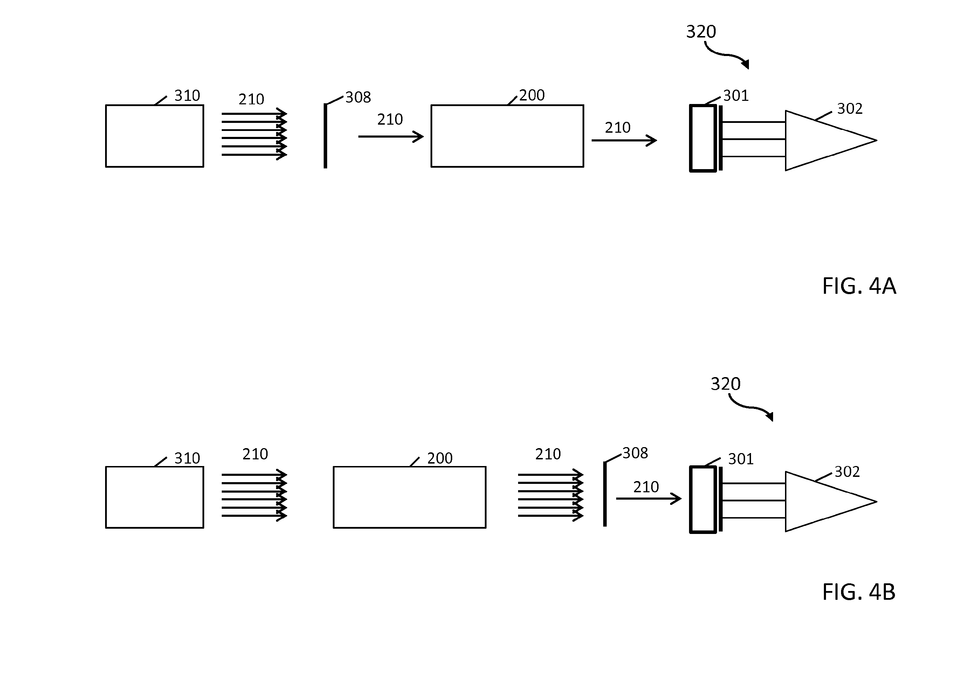

FIG. 4A shows an illustrative embodiment of a sensor for detecting a target of interest (e.g., a biomolecule). A light receiving unit 320 may include a photodetector unit. A light source 310 may emit visible light or ultra-violet light (shown as arrows in FIG. 4A), and a light filter 308 for selecting a light of a specific wavelength from the visible light or ultra-violet light may be disposed between the light source 310 and a apparatus 200 for detecting a target of interest. The light may be selected based on the target of interest. For example, if the target of interest is enterovirus 71, influenza A virus or influenza B virus, the light filter 308 may be configured to select light that has a 560 nm wavelength from the visible light or light that has a 280 nm wavelength from the ultra-violet light. Furthermore, if the target of interest is adenovirus, the light filter 308 may be configured to select light that has a 340 nm wavelength from the visible light or light that has a 280 nm wavelength from the ultra-violet light.

The light receiving unit 320 may comprise a photodiode chip 301 for receiving the light and a photodetector circuit 302 for measuring the intensity of the selected light that passes through the apparatus 200 for detecting a target of interest and generating an electrical signal that is proportionate to the amount of light received by the light receiving unit 320.

FIG. 4B shows an illustrative embodiment of a sensor for detecting a target of interest (e.g., a biomolecule). The sensor illustrated in FIG. 4B is the same as or similar to the sensor illustrated in FIG. 4A, except the light filter 308 is disposed between the apparatus 200 for detecting a target of interest and the photodiode chip 301, instead of between the light source 310 and the apparatus 200 for detecting a target of interest. Therefore, the light having the wavelength specific to the target is selected after the light has passed through the apparatus 200 for detecting a target of interest.

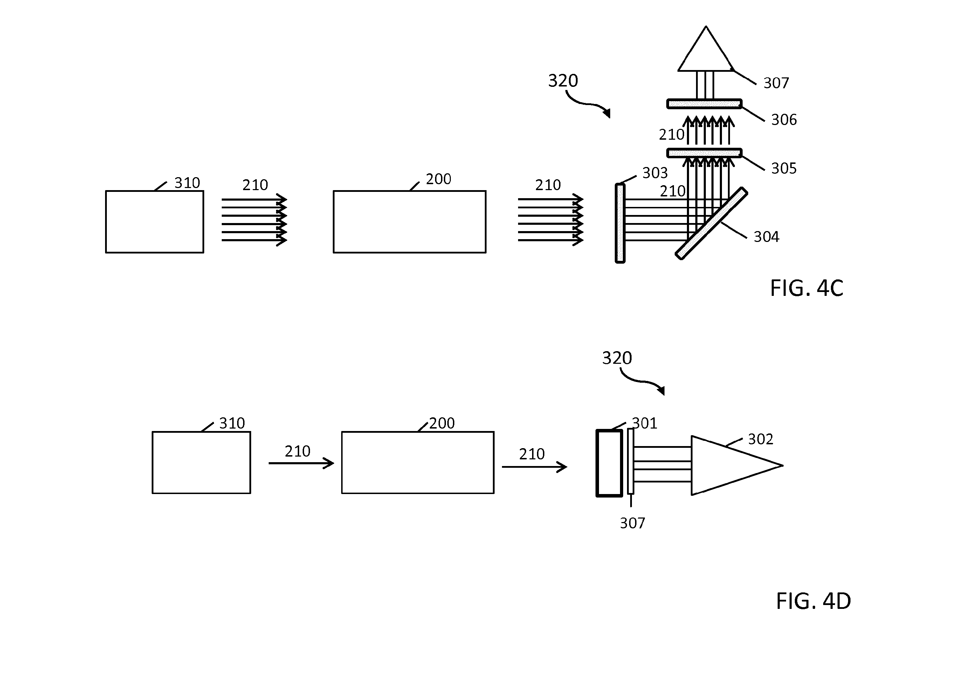

FIG. 4C shows an illustrative embodiment of a sensor for detecting a target of interest (e.g., a biomolecule). The sensor illustrated in FIG. 4C is the same as or similar to the sensor illustrated in FIG. 4A, except that the light filter 308 and photodiode chip 301 are eliminated and the light receiving unit 320 in FIG. 4C comprises a slit 303, a mirror 304, a grating 305, and a linear charge-coupled device (CCD) 306. The visible light that passes through the apparatus 200 for detecting a target of interest enters the slit 303. The grating 305 separates lights of different wavelengths, which are then received by the linear CCD 306, and the photodetector circuit 302 is configured to measure the intensity of the light having the desired wavelength.

FIG. 4D shows an illustrative embodiment of a sensor for detecting a target of interest (e.g., a biomolecule). The sensor illustrated in FIG 4D is the same as the sensor illustrated in FIG. 4A, except that the light source 310 in FIG. 4D emits a monochromatic light having a specific wavelength associated with the target of interest. Therefore, the light filter 308 may be eliminated in the example illustrated in FIG. 4D. The light receiving unit 320 may further comprise an amplifier 307 for amplifying signals received by the photodiode chip 301.

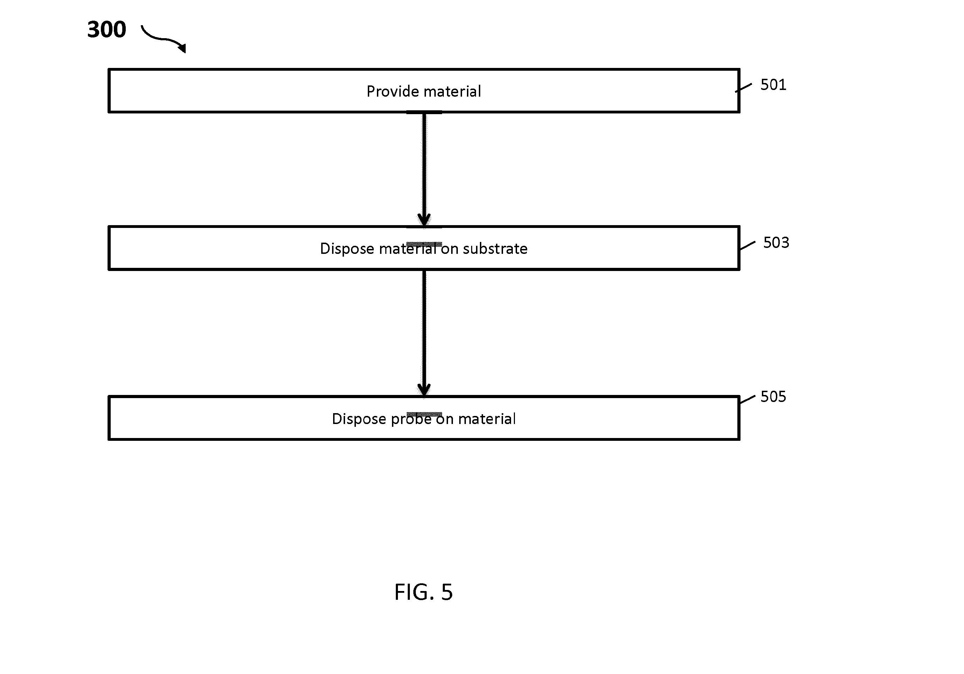

Fig. 5 shows a flow chart of an illustrative embodiment of a method 500 for making an apparatus for binding a target of interest. The method 500 includes steps 501, 503 and 505. In step 501, a material is provided. In step 503, the material is disposed on a substrate. The material may be disposed on the substrate in a pattern configured to increase the surface area available for disposing probes. The pattern may be, for example, a film, a film with an uneven thickness, or a rod array. In step 505, a probe that is capable of binding to a target of interest is disposed on the material. The probe is configured to interact with the target that the sensor configured to detect. In some embodiments, before disposing the probe on the material, the material may be cleaned and pre-treated. For example, the material may be cleaned with an acidic solution, a basic solution, and/or purified water. In some embodiments, the material may be pre-treated with one or more compounds. In one embodiment, the material may be pretreated with one or more compound(s) that include(s) at least one functional group compatible with the material. In another embodiment, the material may be pretreated with one or more compound(s) that include(s) at least one functional group compatible with the probe. The functional group is configured to form a first stable bound with the free electrons around the surface of the material and form a second stable bound with the probe. Some example functional groups include, but not limited to, thiol group and hydroxyl group.

### [Example 1]

### [Probe Immobilization]

A glass container configured to contain a sample was placed in a plastic holder. Gold was disposed on an inner surface of the container in the form of a film having an uneven thickness which varies from approximately 0.5nm to approximately 30nm. Before introducing probes into the container, the gold film was cleaned with a 0.1M hydrochloric acid solution, purified water, 0.1M sodium hydroxide, and purified water, in sequence.

After cleaning, an aqueous solution containing 110µL of cystamine (20mM in phosphate buffered saline (PBS) solution at pH 7.2) was added into the container and incubated for 20 minutes at room temperature to permit cystamine to bind to the gold on the container wall. The remaining cystamine solution was then removed from the container by a pipette. An aqueous solution containing 110µL of glutaraldehyde (2.5% in PBS solution at pH 7.2) was then added into the container and incubated for 20 minutes at room temperature to permit glutaraldehyde to bind to the cystamine.

After removing the remaining glutaraldehyde solution from the container, an aqueous solution of 110µL of commercially available anti-Enterovirus 71 monoclonal antibodies was added into the container and incubated for 20 minutes at room temperature to permit anti-Enterovirus 71 monoclonal antibodies to bind to the glutaraldehyde crosslinker. Unbound anti-Enterovirus 71 monoclonal antibody was then removed from the container by a pipette. An aqueous solution of 0.5M glycine was then added to the container to react with residual unbound glutaraldehyde. Finally, glycine was removed from and PBS was added to the container.

### [Sample Detection]

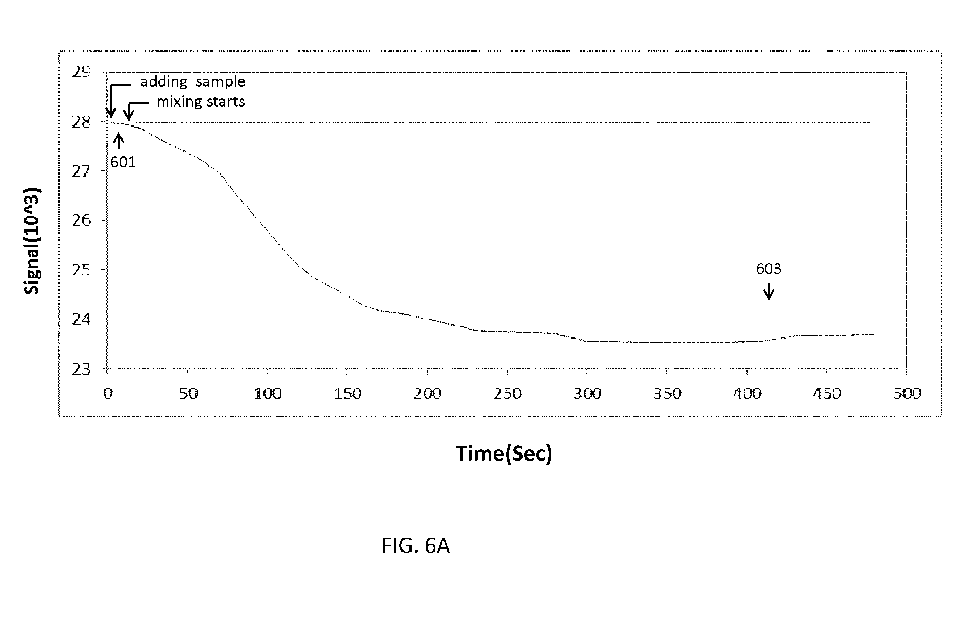

The glass container and the plastic holder were then placed in *pTricorder*® sensor (Vsense Medtech, Taipei, Taiwan). The sensor further includes a visible light source and a light detector for detection of Enterovirus 71. Visible light was transmitted from the visible light source and passed through an optical filter. The optical filter was configured to filter the visible light and only the light having a 560 nm wavelength can pass the optical filter. The light (i.e., having the wavelength of 560 nm) then passed through the glass container set forth above. After passing through the glass container, the light was eventually received by the light detector. The sensor further includes a circulation means configured to circulate the substances in the container, so as to propel Enterovirus 71, if any, towards the probe. FIG. 6A is a chart illustrating the signal strength during detection of Enterovirus 71 in a 10% diluted sample collected from an infected patient. The sample was collected by a throat swab from the infected patient.

The sensor was turned on so that light transmitted from the light source of the sensor passed through the lower portion of the container, without passing through the probes on the inner surface of the container. At this stage, the container contained PBS as set forth above.

After the sample was added, data was collected for generating a signal corresponding to the light detected from the container. The signal detected with PBS and the sample (as shown at 601 in FIG. 6A) was used as a reference. Subsequently, the circulation means was turned on for 3 minutes, allowing the sample in the container to be propelled towards the probes on the inner surface of the container. Data was collected for another 5 minutes. Light detected from the container after the circulation means had stopped is shown at 603 in FIG. 6A. The difference between the light signal detected at 601 and the signal detected at 603 indicated presence of Enterovirus 71 in the sample.

### [Example 2]

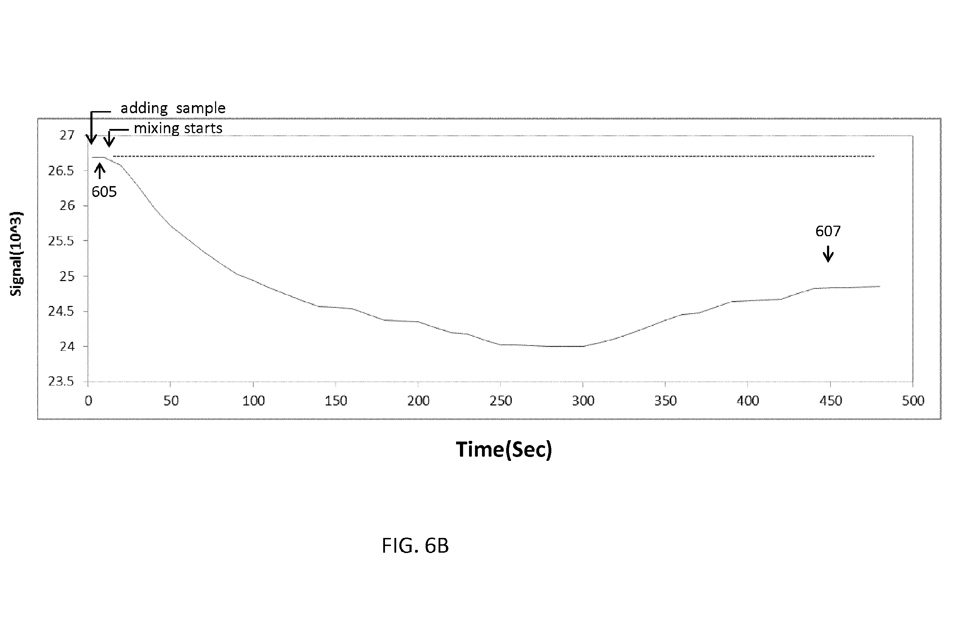

FIG. 6B is a chart illustrating the signal strength during detection of Enterovirus 71 in a 10% diluted sample collected from an infected patient. The detection approach was the same as the approach of the detection of the 10% Enterovirus 71 diluted sample set forth above.

The sensor was turned on so that light transmitted from the light source of the sensor passed through the lower portion of the container, without passing through the probes on the inner surface of the container. At this stage, the container contained PBS as set forth above.

After the sample was added, data was collected for generating a signal corresponding to the light detected from the container. The signal detected with PBS and the sample (as shown at 605 in FIG. 6B) was used as a reference. Subsequently, the circulation means was turned on for approximately 3 minutes, allowing the sample in the container to be propelled towards the probes on the inner surface of the container. Data was collected for another 5 minutes. Light detected from the container after the circulation means had stopped is shown at 607 in FIG. 6B. The difference between the light signal detected at 605 and the signal detected at 607 indicated presence of Enterovirus 71 in the sample.

### [Comparative Example 1]

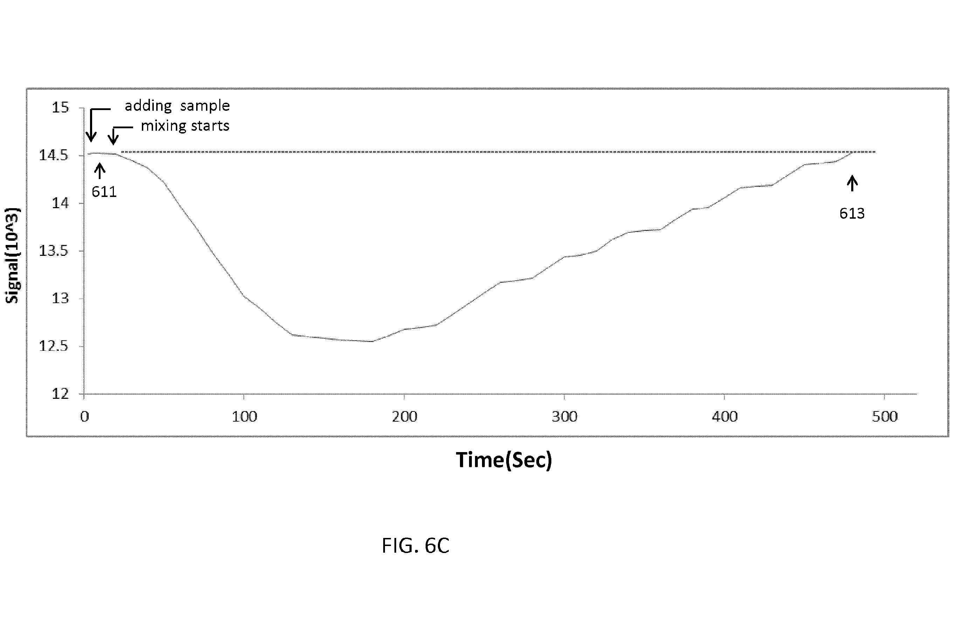

FIG. 6C is a chart illustrating the signal strength during detection of a control sample collected from a healthy person. The detection approach was the same as the approach of the detection of the 10% Enterovirus 71 diluted sample set forth above.

The sensor was turned on so that a light transmitted from the light source of the sensor passed through a lower portion of the container, without passing through the probes on the inner surface of the container. At this stage, the container contained PBS.

After the sample was added, data was collected for generating a signal corresponding to the light detected from the container. The signal detected with PBS and the sample (as shown at 611 in FIG. 6C) was used as a reference. Subsequently, the circulation means was turned on for approximately 3 minutes, allowing the sample in the container to be propelled towards the probes on the inner surface of the container. Data was collected for another 5 minutes. Light detected from the container after the circulation means had stopped is shown at 613 in FIG. 6C. The similar signal strength of 611 and 613 showed no existence of Enterovirus 71 in the control sample.

### [Example 3]

### [Probe Immobilization]

A glass container configured to contain a sample was placed in a plastic holder. Gold was disposed on an inner surface of the container in the form of a film having a substantially even thickness of approximately 100nm. Before introducing probes into the container, the gold film was cleaned with a 0.1M hydrochloric acid solution, purified water, 0.1M sodium hydroxide, and and purified water, in sequence.

After cleaning, an aqueous solution containing 110µL of cystamine (20mM in phosphate buffered saline (PBS) solution at pH 7.2) was added into the container and incubated for 20 minutes at room temperature to permit cystamine to bind to the gold on the container wall. The remaining cystamine solution was then removed from the container by a pipette. An aqueous solution containing 110µL of glutaraldehyde (2.5% in PBS solution at pH 7.2) was then added into the container and incubated for 20 minutes at room temperature to permit glutaraldehyde to bind to the cystamine.

After removing the remaining glutaraldehyde solution from the container, an aqueous solution of 110µL of commercially available anti-Influenza A antibody (20µg/ml in PBS solution at pH 7.2) was added into the container and incubated for 20 minutes at room temperature to permit the anti-Influenza A antibody to bind to the glutaraldehyde crosslinker. Unbound anti-Influenza A antibody was then removed from the container by a pipette. An aqueous solution of 0.5M glycine was then added to the container to react with residual unbound glutaraldehyde. Finally, glycine was removed from and PBS was added to the container.

### [Sample Detection]

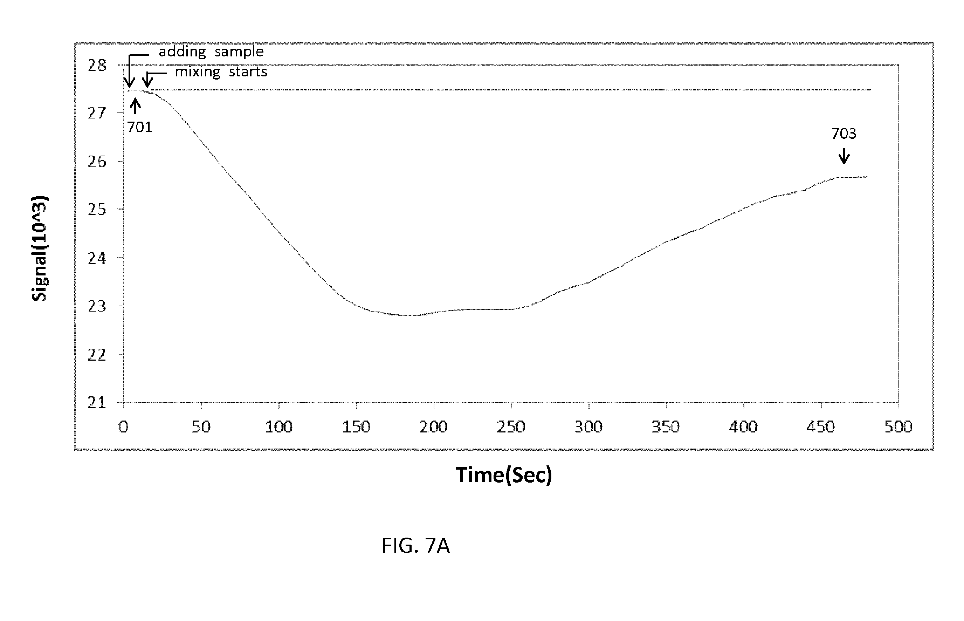

The glass container and the plastic holder were then placed in *pTricorder*® sensor (Vsense Medtech, Taipei, Taiwan). The sensor further includes a visible light source and a light detector for detection of Influenza A. Visible light was transmitted from the visible light source and passed through an optical filter. The optical filter was configured to filter the visible light and only the light having a 560 nm wavelength can pass the optical filter. The light (i.e., having the wavelength of 560 nm) then passed through the glass container set forth above. After passing through the glass container, the light was eventually received by the light detector. The sensor further includes a circulation means configured to circulate the substances in the container, so as to propel Influenza A, if any, towards the probe. FIG. 7A is a chart illustrating the signal strength during detection of Influenza A in a 10% diluted sample collected from an infected patient. The sample was collected by a throat swab from the infected patient's throat.

The sensor was turned on so that light transmitted from the light source of the sensor passed through the lower portion of the container, without passing through the probes on the inner surface of the container. At this stage, the container contained PBS as set forth above.

After the sample was added, data was collected for generating a signal corresponding to the light detected from the container. The signal detected with PBS and the sample (as shown at 701 in FIG. 7A) was used as a reference. Subsequently, the circulation means was turned on for approximately 3 minutes, allowing the sample in the container to be propelled towards the probes on the inner surface of the container. Data was collected for another 5 minutes. Light detected from the container after the circulation means had stopped is shown at 703 in FIG. 7A. The difference between the light signal detected at 701 and the signal detected at 703 indicated presence of Influenza A in the sample.

### [Example 4]

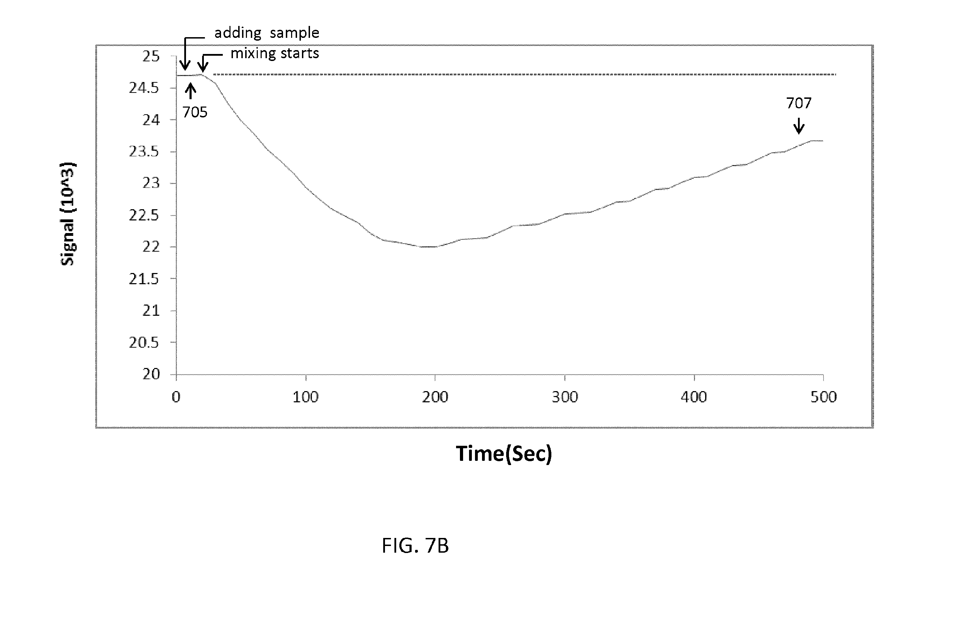

FIG. 7B is a chart illustrating the signal strength during detection of Influenza A in a 10% diluted sample collected from an infected patient. The detection approach was the same as the approach of the detection of the 10% Influenza A diluted sample set forth above.

The sensor was turned on so that light transmitted from the light source of the sensor passed through the lower portion of the container, without passing through the probes on the inner surface of the container. At this stage, the container contained PBS as set forth above.

After the sample was added, data was collected for generating a signal corresponding to the light detected from the container. The signal detected with PBS and the sample (as shown at 705 in FIG. 7B) was used as a reference. Subsequently, the circulation means was turned on for approximately 3 minutes, allowing the sample in the container to be propelled towards the probes on the inner surface of the container. Data was collected for another 5 minutes. Light detected from the container after the circulation means had stopped is shown at 707 in FIG. 7B. The difference between the light signal detected at 705 and the signal detected at 707 indicated presence of Influenza A in the sample.

### [Comparative Example 2]

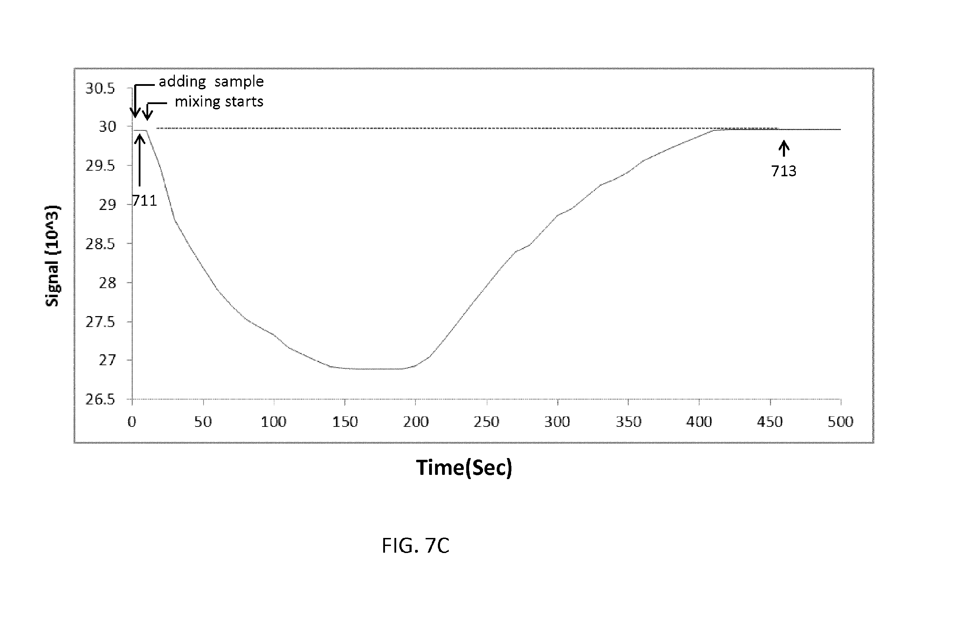

FIG. 7C is a chart illustrating the signal strength during detection of a control sample collected from a healthy person. The detection approach was the same as the approach of the detection of the Influenza A diluted sample set forth above.

The sensor was turned on so that a light transmitted from the light source of the sensor passed through a lower portion of the container, without passing through the probes on the inner surface of the container. At this stage, the container contained PBS.

After the sample was added, data was collected for generating a signal corresponding to the light detected from the container. The signal detected with PBS and the sample (as shown at 711 in FIG. 7C) was used as a reference. Subsequently, the circulation means was turned on for approximately 3 minutes, allowing the sample in the container to be propelled towards the probes on the inner surface of the container. Data was collected for another 5 minutes. Light detected from the container is shown at 713 in FIG. 7C. The similar signal strength of 711 and 713 showed no existence of Influenza A in the control sample.

### [Example 5]

### [Probe Immobilization]

A glass container configured to contain a sample was placed in a plastic holder. Gold was disposed on an inner surface of the container in the form of a film having a pattern of a rod array. A rod of the rod array has a length of approximately 500 nm, a width of approximately 500 nm and a height of approximately 100 nm, and each rod is spaced apart from another rod by apparoximately 500 nm.. Before introducing probes into the container, the gold film was cleaned with a 0.1M hydrochloric acid solution, purified water, 0.1M sodium hydroxide, and and purified water, in sequence.

After cleaning, an aqueous solution containing 110µL of cystamine (20mM in phosphate buffered saline (PBS) solution at pH 7.2) was added into the container and incubated for 20 minutes at room temperature to permit cystamine to bind to the gold on the container wall. The remaining cystamine solution was then removed from the container by a pipette. An aqueous solution containing 110µL of glutaraldehyde (2.5% in PBS solution at pH 7.2) was then added into the container and incubated for 20 minutes at room temperature to permit glutaraldehyde to bind to the cystamine.

After removing the remaining glutaraldehyde solution from the container, an aqueous solution of 110µL of commercially available anti-Influenza B antibody (20µg/ml in PBS solution at pH 7.2) was added into the container and incubated for 20 minutes at room temperature to permit the anti-Influenza B antibody to bind to the glutaraldehyde crosslinker. Unbound anti-Influenza B antibody was then removed from the container by a pipette. An aqueous solution of 0.5M glycine was then added to the container to react with residual unbound glutaraldehyde. Finally, glycine was removed from and PBS was added to the container.

### [Sample Detection]

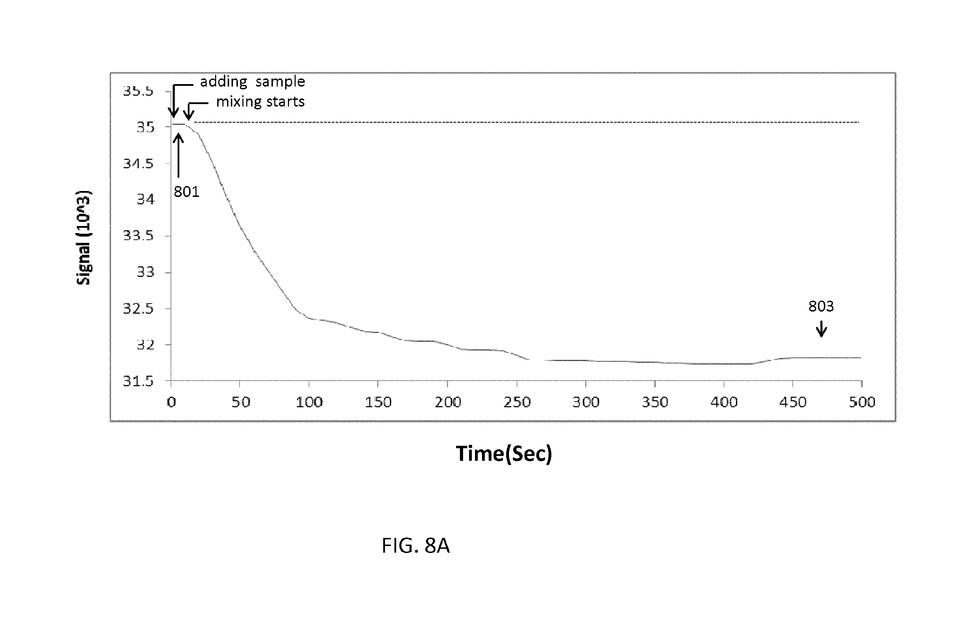

The glass container and the plastic holder were then placed in *pTricorder*® sensor (Vsense Medtech, Taipei, Taiwan). The sensor further includes a visible light source and a light detector for detection of Influenza B. Visible light was transmitted from the visible light source and passed through an optical filter. The optical filter was configured to filter the visible light and only the light having a 560 nm wavelength can pass the optical filter. The light (i.e., having the wavelength of 560 nm) then passed through the glass container set forth above. After passing through the glass container, the light was eventually received by the light detector. The sensor further includes a circulation means configured to circulate the substances in the container, so as to propel Influenza B, if any, towards the probe. FIG. 8A is a chart illustrating the signal strength during detection of Influenza B in a 10% diluted sample collected from an infected patient. The sample was collected by a throat swab from the infected patient's throat.

The sensor was turned on so that light transmitted from the light source of the sensor passed through the lower portion of the container, without passing through the probes on the inner surface of the container. At this stage, the container contained PBS as set forth above.

After the sample was added, data was collected for generating a signal corresponding to the light detected from the container. The signal detected with PBS and the sample (as shown at 801 in FIG. 8A) was used as a reference. Subsequently, the circulation means was turned on for approximately 3 minutes, allowing the sample in the container to be propelled towards the probes on the inner surface of the container. Data was collected for another 5 minutes. Light detected from the container after the circulation means had stopped is shown at 803 in FIG. 8A. The difference between the light signal detected at 801 and the signal detected at 803 indicated presence of Influenza B in the sample.

### [Example 6]

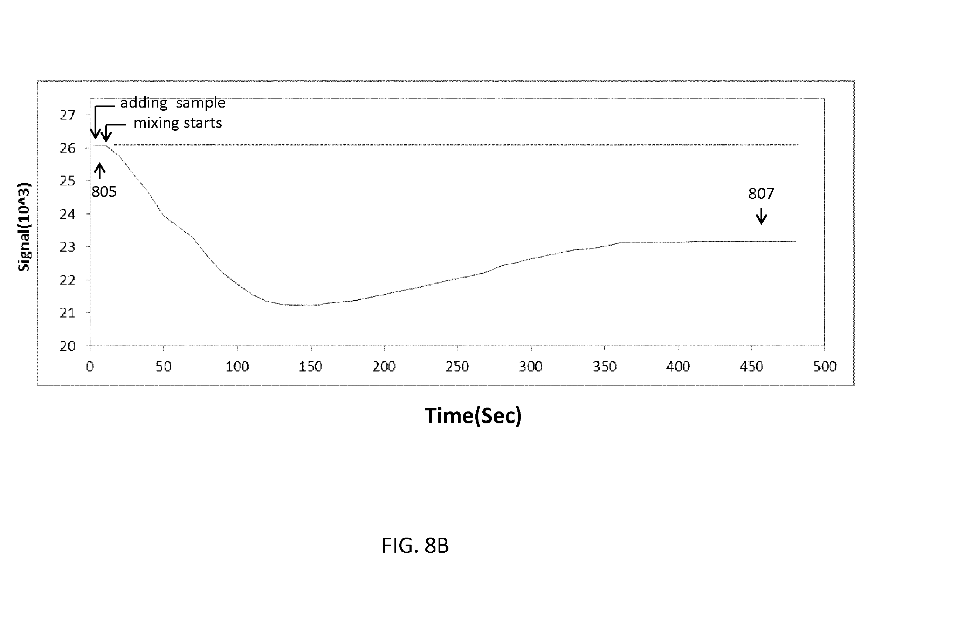

FIG. 8B is a chart illustrating the signal strength during detection of Influenza B in a 10% diluted sample collected from an infected patient. The detection approach was the same as the approach of the detection of the 10% Influenza B diluted sample set forth above.

The sensor was turned on so that light transmitted from the light source of the sensor passed through the lower portion of the container, without passing through the probes on the inner surface of the container. At this stage, the container contained PBS as set forth above.

After the sample was added, data was collected for generating a signal corresponding to the light detected from the container. The signal detected with PBS and the sample (as shown at 805 in FIG. 8B) was used as a reference. Subsequently, the circulation means was turned on for approximately 3 minutes, allowing the sample in the container to be propelled towards the probes on the inner surface of the container. Data was collected for another 5 minutes. Light detected from the container after the circulation means had stopped is shown at 807 in FIG. 8B. The difference between the light signal detected at 805 and the signal detected at 807 indicated presence of Influenza B in the sample.

### [Comparative Example 3]

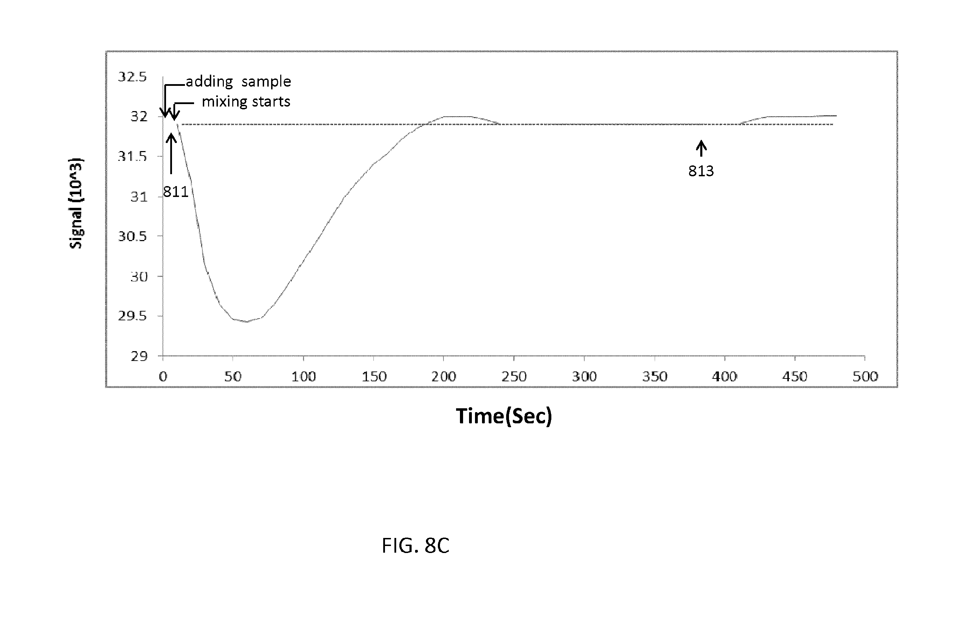

FIG. 8C is a chart illustrating the signal strength during detection of a control sample collected from a healthy person. The detection approach was the same as the approach of the detection of the Influenza B diluted sample set forth above.

The sensor was turned on so that a light transmitted from a light source of the sensor passed through a lower portion of the container, without passing through the probes on the inner surface of the container. At this stage, the container contained PBS. The signal detected with PBS (as shown at 811 in FIG. 8C) was used as a reference.

After the sample was added, the circulation means was turned on for approximately 3 minutes, allowing the sample in the container be propelled towards the probes on the inner surface of the container. Data was collected for another minutes. Light detected from the container after the circulation means had stopped is shown at 813 in FIG. 8C. The similar signal strength of 811 and 813 showed no existence of Influenza B in the control sample.

Although the foregoing invention has been described in some detail by way of illustration and examples for purposes of clarity of understanding, it will be apparent to those skilled in the art that certain changes and modifications may be practiced without departing from the spirit and scope of the invention. Therefore, the description should not be construed as limiting the scope of the invention.

All publications, patents, and patent applications cited herein are hereby incorporated by reference in their entireties for all purposes and to the same extent as if each individual publication, patent, or patent application were specifically and individually indicated to be so incorporated by reference.

## Claims

1. A sensor comprising:
a container;
a probe disposed in the container and configured to bind to a target of interest;
a circulation means configured to circulate substances in the container;
a light source;
a light receiver;
a light selecting unit for allowing light of a predetermined wavelength be received by the light receiver; and
a detector configured to generate an electrical signal, the magnitude of which reflects the amount of light that is received by the light receiver,
wherein the container is located between the light source and the light receiver, and
wherein the container is configured such that the probe and the path of the light emitted by the light source are in different regions inside the container.

2. The sensor of claim 1, wherein the light receiver includes the light selecting unit, which comprises:
a slit for receiving the light, a mirror, a grating, and a linear charge-coupled device ,
wherein the mirror is configured to reflect the received light onto the grating, the grating is configured to separate the received light into a plurality of lights of different wavelengths and transmit the plurality of lights to the linear charge-coupled device, and
wherein the detector comprises a photodetector circuit for measuring the intensity of one of the plurality of light received by the light receiver and generating the electrical signal that is proportionate to the intensity of the one of the plurality of light, the one of the plurality of light has the predetermined wavelength.

3. The sensor of claim 1, wherein the light receiver comprises a photodiode chip for receiving the light, and
wherein the detector comprises a photodetector circuit for measuring the intensity of the light received by the light receiver and generating the electrical signal that is proportionate to the intensity of the light received by the light receiver.

4. The sensor of claim 3, wherein the light selecting unit comprises a light filter disposed between the light source and the container or between the container and the light receiver.

5. The sensor of one of the preceding claims further comprises an amplifier for amplifying signals received by the light receiver, and/or
wherein the light emitted from the light source comprises a wavelength of about 200 nm to about 800 nm, and/or
wherein the predetermined wavelength is associated with the target of interest.

6. The sensor of one of the preceding claims, wherein the probe is disposed in an upper portion of the container and the path of the light is in a lower portion of the container.

7. The sensor of one of claims 1 to 5, wherein the probe is disposed in a first region in an upper portion of the container and the path of the light is in a second region, different from the first region, in the upper portion of the container.

8. The sensor of one of the preceding claims, wherein the probe comprises DNA, RNA, a protein, an antibody, an antibody fragment, an aptamer, an antigen, or an epitope, and/or
wherein the target of interest is a biomolecule.

9. The sensor of one of the preceding claims, wherein the target comprises a virus, a protein, a nucleic acid, a carbohydrate, a lipid, a hapten, or a toxin, and/or
wherein the probe binds to the target of interest with an affinity of about 100 pN to about 500 pN.

10. The sensor of one of the preceding claims, wherein the circulation means further comprises:
a propelling means disposed in the container; and
a motor electrically connected to the propelling means and configured to actuate the propelling means.

11. The sensor of one of claims 1 to 9, wherein the circulation means further comprises:
a first magnet freely disposed in the container; and
a motor, which includes a second magnet, configured to actuate the first magnet.

12. The sensor of one of the preceding claims, wherein the container further comprises:
a substrate; and
a material disposed on the substrate;
wherein the probe is disposed on the material.

13. The sensor of claim 12, wherein the material comprises a metal.

14. The sensor of claim 12 or 13, wherein the material comprises a film on the substrate.

15. The sensor of claim 14, wherein the film comprises a substantially even thickness or an uneven thickness, in particular a substantially even thickness is in the range of 5 nm to 200 nm or an uneven thickness varies from 0.5 nm to 30 nm.

16. The sensor of claim 12 or 13, wherein the material comprises a rod array disposed on the substrate.

17. The sensor of claim 16, wherein the size of a rod in the rod array is associated with the size of the probe and the target of interest, in particular a rod of the rod array has a length from 200 to 900 nm, a width from 200 to 900 nm and a height from 15 to 1500 nm.

18. A sensor comprising:
a container;
a probe disposed in the container and configured to bind to a target of interest;
a circulation means configured to circulate substances in the container;
a light source that emits light of a predetermined wavelength;
a light receiver; and
a detector configured to generate an electrical signal, the magnitude of which reflects the amount of light that is received by the light receiver,
wherein the container is located between the light source and the light receiver, and
wherein the container is configured such that the probe and the path of the light emitted by the light source are in different regions inside the container.
